# FASCICULE DE BREVET EUROPEEN

(11) **EP 3 068 515 B1**
(45) Date de publication et mention de la délivrance du brevet: **30.08.2017**
(21) Numéro de dépôt: 14821694.8
(22) Date de dépôt: 13.11.2014
(51) Int. Cl.: B01D 39/20

(54) **NOUVEAU MILIEU FILTRANT POUR L'OBTENTION DE PLASMA, DISPOSITIF ET PROCÉDÉ DE FILTRATION ASSOCIÉS**
NEUARTIGES FILTERMEDIUM ZUR GEWINNUNG VON PLASMA SOWIE ZUGEHÖRIGE FILTRATIONSVORRICHTUNG UND VERFAHREN
NOVEL FILTER MEDIUM FOR OBTAINING PLASMA, AND ASSOCIATED FILTRATION DEVICE AND METHOD

(30) Priorité: 14.11.2013 FR 1361140
(43) Date de publication de la demande: 21.09.2016
(73) Titulaire: Biomérieux, 69280 Marcy l'Étoile (FR)
(72) Inventeur: FOUCAULT, Frédéric, F-69280 Marcy l'Etoile (FR)
(74) Mandataire: Sarlin, Laure V.
(86) Numéro de dépôt international: PCT/FR2014/052889
(87) Numéro de publication internationale: WO 2015/071597

(56) Documents cités:
- EP-A1- 0 406 485
- EP-A1- 0 606 646
- EP-A2- 0 392 377
- WO-A1-2004/018078

## Description

La présente invention concerne le domaine technique du traitement du sang, et notamment de l'extraction du plasma à partir d'un échantillon de sang, et en particulier d'un échantillon de sang total.

Plus précisément, l'invention concerne un milieu filtrant, un système de filtration et un dispositif pour transférer une partie d'un échantillon de sang, contenu dans un récipient muni d'un bouchon contenant un tel milieu filtrant, ainsi que les procédés associés,

Dans le domaine des analyses biologiques à partir d'échantillons de sang, il est largement répandu de récupérer un échantillon de sang dans un récipient se présentant sous la forme d'un tube obturé par un bouchon tel qu'un septum pénétrable par une aiguille creuse. De nombreuses solutions ont été proposées pour obtenir du plasma à partir d'un tel échantillon de sang total Ces solutions reposent entre autres sur deux techniques possibles pour extraire du plasma à partir du sang, à saloir la filtration et la centrifugation.

L'isolation du plasma sanguin, par centrifugation, nécessite la mise en oeuvre d'un appareillage complexe et onéreux pour, d'une part, séparer les composants du sang en fonction de leur différence de densité en les soumettant à une force centrifuge et d'autre part, extraire le plasma de la partie du sang contenant les hématies (les globules rouges) ayant eu le temps de sédimenter, Indépendamment de la nécessité d'avoir recours à une centrifugeuse et à des tubes à centrifugation, cette technique présente un temps de centrifugation relativement long, avec une difficulté pour définir la force centrifuge pour obtenir un plasma dépourvu des éléments figurés du sang.

L'extraction du plasma par filtration nécessite l'utilisation d'un milieu de séparation pour le sang. L'article « Micro-scale blood plasma réparation: from acoustophoresis to egg-beaters », publié dans 'The Royal Society of Chemistry 2013', fait une revue des diverses solutions connues pour assurer une séparation du plasma à l'échelle microscopique, Parmi ces techniques, on peut citer celles décrites dans les documents suivants :
- le document EP 0 392 377 décrit un dispositif permettant de séparer le plasma ou sérum à partir d'un échantillon de sang total, mettant en oeuvre un filtre comprenant un agent d'agglutination des cellules sanguines ;
- les documents WO 2004/018078, EP 0 406 485 et EP 0 606 646, quant à eux, décrivent des filtres permettant d'éliminer les leucocytes d'échantillons de sang, correspondant à des filtres de structure asymétrique.

Le sang humain présente un taux d'hématocrite variable, suivant l'activité et la santé du sujet, notamment en cas d'anémie ou de polyglobulie, Plus le taux d'hématocrite est élevé, plus l'opération de filtration est difficile, En particulier, des problèmes de bouchage/colmatage, d'hémolyse, de faible vitesse d'extraction sont rencontrés.

L'analyse des techniques antérieures connues conduit à constater qu'il n'existe pas de solution simple, conduisant à une filtration satisfaisante, ce à moindre coût et avec un minimum d'opérations, en limitant les problèmes de bouchage/colmatage. En particulier, l'Invention se propose de fournir un milieu filtrant permettant d'améliorer significativement la filtration du sang pour l'obtention de plasma, en termes de quantité (volume plus élevé en un temps donné) et en termes de qualité (moins d'hémolyse et meilleure élimination des éléments figurés du sang).

Dans ce contexte, la présente invention concerne un milieu filtrant comprenant deux zones poreuses positionnées sous la forme d'un empilement :
- une première zone poreuse, dite de préfiltration, traitée avec au moins un réactif présentant une affinité pour les hématies et entrainant la capture ou l'agglutination des hématies sur la zone de préfiltration,
- une deuxième zone poreuse, dite asymétrique, présentant une porosité décroissante transversalement à son épaisseur, la partie de porosité plus élevée étant positionnée du côté de la zone de préfiltration.

La présente invention a également pour objet un système de filtration du sang, et en particulier de sang total, permettant de récupérer du plasma comprenant une chambre intégrant un milieu filtrant selon l'invention et équipée d'une entrée pour le sang à filtrer positionnée du côté de la zone de préfiltration et d'une sortie pour le plasma située du côté de la zone asymétrique, le milieu filtrant étant positionné dans la chambre de manière à ce que le sang circule depuis l'entrée pour traverser transversalement les deux zones du milieu filtrant, et récupérer le plasma à la sortie.

Un autre objet de l'invention concerne un procédé d'obtention de plasma à partir d'un échantillon de sang, et en particulier d'un échantillon de sang total, mettant en oeuvre un système selon l'invention dans lequel l'échantillon de sang est introduit dans le système par l'entrée et le plasma est récupéré à la sortie.

Il existe également un besoin pour une nouvelle technique permettant, sans risque de contamination, d'extraire relativement rapidement, d'un récipient obturé contenant un prélèvement de sang, et en particulier un prélèvement de sang total, une quantité non négligeable de plasma,

L'objet de l'invention vise donc à satisfaire ce besoin en proposant une nouvelle technique pour extraire simplement sans risque de contamination, et dans un temps relativement court, une partie d'un échantillon de sang contenu dans un récipient muni d'un bouchon.

Aussi, l'invention concerne, également, un dispositif pour transférer une partie d'un échantillon de sang, et en particulier d'un échantillon de sang total, contenu dans un récipient muni d'un bouchon, et conduisant à la récupération de plasma, caractérisé en ce qu'il comprend :
- une aiguille creuse pourvue d'une extrémité distale de pénétration à travers le bouchon du récipient et d'une extrémité proximale,
- une chambre de pressurisation d'un fluide traversée par l'extrémité proximale de l'aiguille creuse de transfert et équipée d'un septum positionné pour être pénétrable par l'extrémité proximale de l'aiguille après déplacement de cette dernière, cette chambre étant étanche et séparée par le septum, d'une chambre de distribution du liquide dans laquelle peut déboucher l'extrémité proximale de l'aiguille creuse, est
- un système de rapprochement relatif entre l'aiguille creuse et le septum pour assurer une augmentation de la pression du fluide à la suite dudit rapprochement, ce système de rapprochement étant commandé selon une course déterminée pour dans une première phase, augmenter la pression à l'intérieur de la chambre étanche jusqu'à atteindre une pression de transfert juste avant le passage de l'aiguille creuse à travers le septum et dans une deuxième phase, que l'aiguille creuse traverse le septum pour déboucher dans la chambre de distribution du sang possédant une pression inférieure à la pression de transfert afin d'assurer, sous l'effet de cette différence de pression, le transfert d'une partie du sang du récipient dans ladite chambre de distribution, ladite chambre de distribution comportant un système de filtration selon l'invention permettant d'assurer l'extraction du plasma.

Un autre objet de l'invention est de proposer un procédé permettant d'extraire à plusieurs reprises, d'un même récipient, une quantité non négligeable de plasma, à partir d'un échantillon de sang, et en particulier d'un échantillon de sang total, contenu dans un récipient muni d'un bouchon.

L'invention a également pour objet un procédé pour transférer une partie d'un échantillon de sang, et en particulier d'un échantillon de sang total, contenu dans un récipient muni d'un bouchon, permettant de récupérer du plasma, caractérisé en ce qu'il consiste :
- à mettre en communication, à l'aide d'une aiguille creuse traversant le bouchon du récipient, l'intérieur du récipient et une chambre étanche de pressurisation d'un fluide, équipée d'un septum pénétrable par aiguille et présentant un volume variable à la suite du déplacement relatif entre l'aiguille creuse et le septum pénétrable par aiguille,
- à assurer le rapprochement relatif entre l'aiguille creuse et le septum pénétrable par aiguille selon une course déterminée afin d'augmenter la pression à l'intérieur de la chambre et par suite du récipient par le transfert du fluide à travers l'aiguille creuse et d'atteindre une pression de transfert juste avant le passage de l'aiguille creuse à travers le septum,

- à poursuivre le rapprochement relatif entre l'aiguille creuse et le septum pénétrable par aiguille afin que l'aiguille creuse traverse le septum pour déboucher dans une chambre de distribution du sang possédant une pression inférieure à la pression de transfert afin d'assurer, sous l'effet de cette différence de pression, le transfert à travers l'aiguille creuse, d'une partie du sang du récipient dans ladite chambre de distribution, et
- à assurer l'écoulement du sang transféré dans la chambre de distribution, à travers un système de filtration selon l'invention pour assurer l'extraction du plasma.

La description ci-dessous en référence aux Figures annexées permet de mieux comprendre l'invention,
La **Figure 1** est une vue schématique en coupe d'un milieu filtrant selon l'invention.
La **Figure 2** est une vue en perspective montrant un exemple de réalisation du dispositif conforme à l'invention d'extraction d'un échantillon de sang contenu dans un récipient.
La Figure 3 est une vue en coupe élévation montrant le dispositif conforme à dans une position initiale d'extraction.
La **Figure 4** est une vue en coupe élévation montrant le dispositif conforme à l'invention dans une position intermédiaire d'extraction.
La **Figure 5** est une vue à plus grande échelle, en coupe élévation montrant le dispositif conforme à l'invention dans une position d'extraction d'une partie de l'échantillon de sang contenu dans un récipient
La **Figure 6** est une vue en perspective montrant le montage d'un récipient sur le piston du dispositif d'extraction conforme à l'invention.
La **Figure 7** est une vue en coupe du corps de réception du piston du dispositif d'extraction conforme à l'invention.

Selon une caractéristique essentielle, le milieu filtrant selon l'invention comporte deux zones distinctes: une zone dite de préfiltration permettant de bloquer les hématies par un phénomène d'agglutination ou de formation d'agrégats et une zone dite asymétrique.

De manière à remplir son rôle de préfiltration, de manière classique, la taille minimale des pores de la zone de préfiltration sera supérieure à la taille maximale des pores de la zone asymétrique. Dans le cadre de l'invention, la taille de pores pourra être mesurée selon la norme ASTM F 316-80. La zone de préfiltration présente une taille de pores supérieure à la taille des hématies, et de préférence appartenant à la gamme allant de 10 µm à 1 mm ; la zone asymétrique présente une taille de pores dans sa partie de porosité maximale laissant passer les hématies, par exemple, appartenant à la gamme allant de 10 à 100 µm et dans sa partie de porosité minimale une taille de pores inférieure à la taille des cellules sanguines, et notamment des hématies, et de préférence appartenant à la gamme allant de 0,1 à 10 µm.

Le matériau constitutif des différentes zones sera, de préférence, choisi de manière à avoir une capacité de mouillage adaptée à la pénétration et à la circulation du sang. Notamment, on choisira des matériaux présentant une tension superficielle de mouillage critique (CWST de l'anglais «critical wetting surface tension ») adaptée.

La CWST peut être mesurée selon la loi de Young-Dupré. Une goutte de liquide va plus ou moins s'étaler à la surface d'un solide. Ce phénomène s'appelle le mouillage et on appelle angle de contact l'angle entre l'interface fluide/gaz et la surface solide. La mesure de l'angle d'une goutte d'eau est la méthode de référence.

De manière avantageuse, la zone de préfiltration a une tension superficielle de mouillage critique comprise entre 50 et 100.10⁻³ N.m⁻¹ et, préférentiellement, supérieure à 53.10⁻³ N.m⁻¹; ces valeurs concernent, bien entendu, la zone de préfiltration porteuse des réactifs présentant une affinité pour les hématies. Selon un mode de réalisation particulier, la zone de préfiltration est un milieu fibreux. Les fibres traitées lorsqu'elles sont traversées par le sang, capturent par affinité les globules rouges lorsque ceux-ci passent à proximité des fibres. Cette capture complète la filtration physique obtenue par le choix de la taille des pores de la zone de préfiltration qui retient une partie des cellules sanguines en fonction de leur taille. De manière avantageuse, la zone de préfiltration sera composée de fibres de verre, avec une densité de 50 à 300 g.m⁻².

De manière avantageuse, la zone asymétrique a, quant à elle, une tension superficielle de mouillage critique comprise entre 50 et 100.10⁻³ N.m⁻¹ et, préférentiellement, supérieure à 70.10⁻³ N.m⁻¹. La zone asymétrique est, par exemple, constituée d'un matériau poreux choisi parmi les polymères synthétiques, tels que les polysulfones, polycarbonates, polyesters, polyoléfines, polyamines, polyacryliques, polyvinyles et les matériaux inorganiques tels que les verres, céramiques, métaux.

La zone de préfiltration est présente pour retenir les agrégats et les plus gros composants présents dans l'échantillon de sang à filtrer, et en particulier dans un échantillon de sang total. Cette zone de préfiltration va agir en tant que premier milieu filtrant, afin de réduire le taux d'hématocrite initial de l'échantillon de sang. Compte tenu du nombre de globules rouges par ml et de leur facile déformation, on a choisi, dans le cadre de l'invention, une préfiltration chimique qui permet de limiter, voire d'éviter totalement le bouchage ultérieur du filtre asymétrique. Cette préfiltration autorise également la filtration de volumes importants de sang, voire plusieurs opérations successives de filtration d'un même échantillon initial de sang total.

Cette zone de préfiltration utilise un principe de filtration par affinité, puisque des partenaires de liaison spécifiques des hématies sont présents sur le filtre pour capturer ces dernières. La liaison qui intervient entre les réactifs présentant une affinité pour les hématies et les hématies sont des liaisons de faible énergie, du type Van der Walls, électrostatiques, hydrogène ... Les molécules ou réactifs présentant une affinité pour les hématies entraînent la capture ou l'agglutination d'au moins une partie des hématies sur la zone de préfiltration. De telles molécules présentant une affinité pour les hématies peuvent notamment être choisies parmi des anticorps spécifiques des antigènes présents sur les hématies, et des lectines, comme l'hémaglutinine. On pourra notamment utiliser des anticorps spécifiques d'un antigène se trouvant sur les hématies du groupe sanguin du patient dont l'échantillon est à analyser, Les anticorps sont dits spécifiques, quand ils sont capables de se lier de façon exclusive, ou quasi exclusive, à l'antigène cible.

L'accrochage des réactifs présentant une affinité pour les hématies sur la zone de préfiltration pourra être mené par toute technique appropriée. Le traitement du filtre destiné à constituer la zone de préfiltration peut être réalisé par voie chimique, notamment par greffage ou absorption. On peut notamment se référer à Lateral Flow Immunoassay, Editeur Raphael C. Wong I Harley Y. Tse (ISBN; 978-1-58829-908-6) publié en 2009, chapitre 1.4.1.3, pages 12-13. Au préalable, un traitement physique du type plasma, irradiation notamment sous UV, pourra être mené pour faciliter accrochage des réactifs présentant une affinité pour les hématies. Aussi, les réactifs présentant une affinité pour les hématies pourront être adsorbés, liés physiquement ou chimiquement en surface et dans l'épaisseur de la zone de préfiltration. De tels filtres sont notamment décrits dans le document GB 2250342. Le plus souvent, un traitement sera réalisé à l'aide d'une formulation comprenant les réactifs présentant une affinité pour les hématies. D'autres molécules peuvent être utilisées dans la formulation, tels que des agents mouillants comme la BSA et la caséine, servant également à empêcher l'adsorption non spécifique. Une formulation de dépôt contenant une quantité de réactifs présentant une affinité pour les hématies correspondant à une concentration comprise entre 0,1 et 1 mg/ml pourra, par exemple, être utilisée.

Le sang traverse, tout d'abord, la zone de préfiltration, puis la zone asymétrique. Lorsque le sang ainsi préalablement appauvri en hématies par passage au travers de la zone de préfiltration arrive sur la zone dite asymétrique, celle-ci permet alors une extraction plus facile et plus efficace du plasma. De plus, la porosité minimale de la zone dite asymétrique est très faible. La présence d'un gradient décroissant de porosité, à partir de la zone de préfiltration, c'est-à-dire dans le sens de circulation du sang, permet d'éviter l'accumulation des globules rouges restant à la surface d'entrée de la zone asymétrique et des phénomènes d'hémolyses et de bouchage.

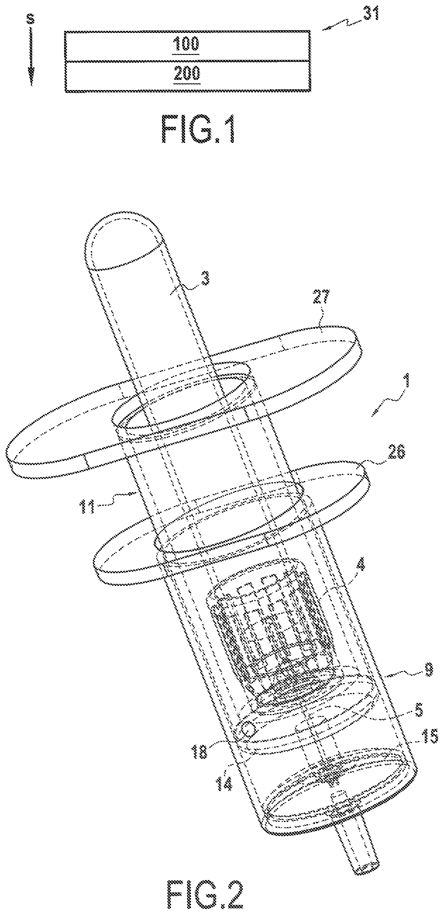

Le plus souvent, comme illustré sur la **Figure 1** présentant schématiquement un milieu filtrant **31,** la zone de préfiltration **100** et la zone asymétrique **200** seront directement en contact l'une de l'autre, bien que la possibilité d'insérer une zone intermédiaire ne soit pas exclue. Au sein de la zone asymétrique **200**, la plus forte porosité se trouve au contact de la zone de préfiltration **100.** Lorsque le sang qui circule selon le sens **s**, ainsi préalablement appauvri en hématies par passage au travers de la zone de préfiltration **100**, arrive sur la zone asymétrique **200,** celle-ci permet alors une extraction plus facile et plus efficace du plasma.

Selon un mode de réalisation particulier, le milieu filtrant est exclusivement constitué de la zone de préfiltration et de la zone asymétrique telles que définies dans le cadre de l'invention. Chacune des zones peut être constituée d'un seul ou de plusieurs éléments filtrants. En particulier, de manière à obtenir l'épaisseur souhaitée, la zone de préfiltration pourra être constituée d'un empilement de plusieurs éléments filtrants traités avec le réactif ou les réactifs présentant une affinité pour les hématies, afin d'obtenir l'épaisseur souhaitée. Il est également envisageable de réaliser la zone de filtration avec au moins un élément filtrant traité avec le réactif présentant une affinité pour les hématies et un ou plusieurs autre(s) éléments) filtrant de porosité adaptée.

De préférence, les différents éléments filtrants constituant la zone de préfiltration seront identiques. En effect on préfèrera une zone de préfiltration, constituée d'un seul ou de plusieurs éléments, mais de taille de pores homogène dans son épaisseur, par opposition à la zone dite asymétrique.

De même, la zone asymétrique pourra être constituée d'un seul élément présentant dans son épaisseur un gradient de porosité, comme ceux commercialisés par la société Pall dans la gamme Vivid^{®}, ou encore d'un empilement de différents éléments de porosité différente et choisis de manière à obtenir la décroissance souhaitée. Dans ce dernier cas, la décroissance de porosité est alors réalisée par pallier.

L'homme du métier ajustera l'épaisseur des différentes zones pour optimiser le ratio porosité/épaisseur/surface et minimiser le volume mort. En général, la zone de préfiltration présentera une épaisseur de 0,3 à 5 mm, et notamment de 1,2 à 3 mm, et/ou la zone asymétrique présente une épaisseur de 0,1 à 5 mm, et notamment de 0,2 à 3 mm.

Le milieu filtrant selon l'invention est particulièrement adapté à la filtration du sang total. De manière classique, on entend par sang total, un prélèvement de sang contenant l'ensemble des constituants du sang (le plasma, les globules blancs et rouges, les plaquettes ...). Un échantillon de sang total peut éventuellement inclure un agent coagulant. Un milieu filtrant pourra notamment être utilisé sur du sang capillaire (goutte de sang récupérée au bout du doigt). En particulier, un tel milieu filtrant pourra être utilisé dans un test réalisé près du patient, tel qu'un test rapide.

En général, le milieu filtrant sera intégré dans un système de filtration du sang, et en particulier de sang total, permettant de récupérer du plasma qui comprendra une chambre intégrant le milieu filtrant et munie d'une entrée pour le sang à filtrer positionnée du côté de la zone de préfiltration et une sortie pour le plasma située du côté de la zone asymétrique, le milieu filtrant étant positionné dans la chambre de manière à ce que le sang circule depuis l'entrée pour traverser transversalement les deux zones du milieu filtrant, et récupérer le plasma à la sortie. Un tel système de filtration 30 pourra lui-même être intégré dans un dispositif d'extraction plus complexe, tel que notamment illustré sur les **Fig. 2** à **7****.** Il est également possible d'envisager d'utiliser le milieu filtrant, selon l'invention, pour aspirer du sang directement sur un patient et extraire le plasma par la suite. Il serait ainsi possible de réaliser des analyses sanguines sur plasma, sans passer par un tube de prélèvement de sang total. Dans ce cas, une pression réduite devrait être appliquée dans le milieu filtrant lui-même, sous la forme d'un vide dynamique ou statique notamment

Le dispositif tel que décrit sur les **Fig. 2** à **7****,** qui constitue l'une des applications majeures du milieu filtrant selon l'invention, va maintenant être décrit en détails. Tel que cela ressort plus précisément des **Fig**. **2** et **3****,** un tel dispositif 1 est conçu pour transférer une partie d'un échantillon de sang **2** contenu dans un récipient **3** se présentant classiquement sous la forme d'un tube fermé hermétiquement par un bouchon **4.** Le récipient **3** sera, en général, un tube à prélèvement de sang sous vide.

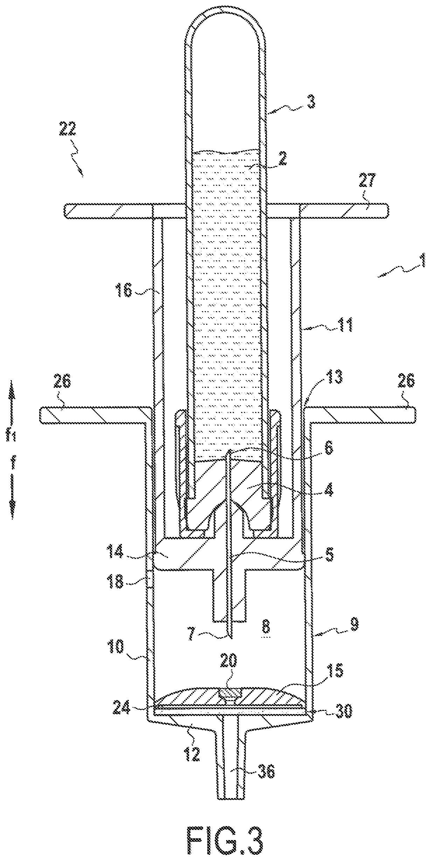

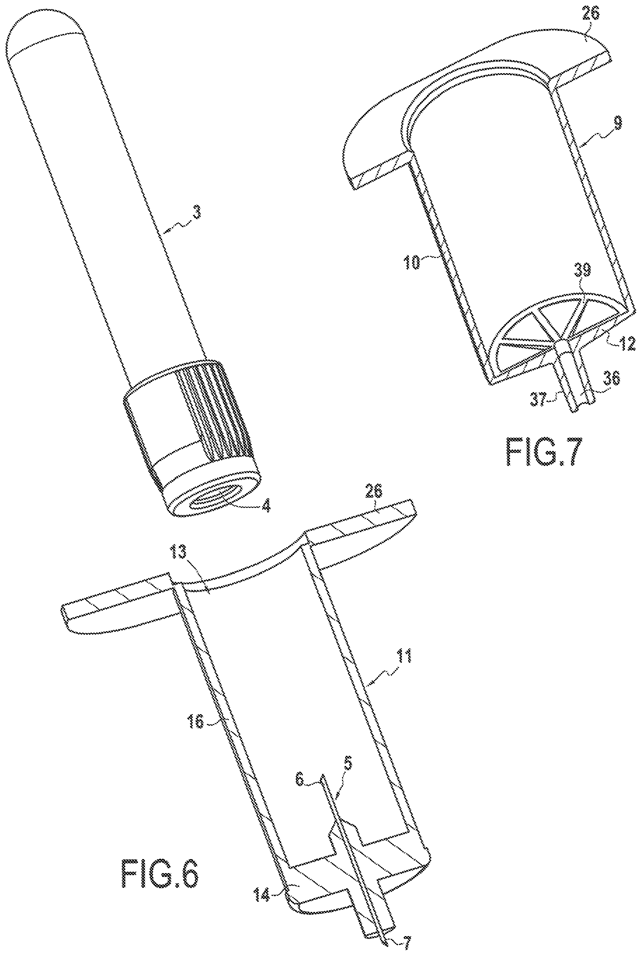

Selon une caractéristique du dispositif **1** selon l'invention, le récipient **3** est apte à être traversé par une aiguille creuse **5** à travers laquelle est transférée ou extraite une partie du sang contenu à l'intérieur du récipient. Le bouchon **4** tel qu'un septum d'un tube de prélèvement est pénétrable par l'aiguille creuse **5.** Tel que cela ressort, plus précisément, des **Fig. 3** et **6****,** l'aiguille creuse **5** est pourvue d'une extrémité distale **6** de pénétration à travers le bouchon **4** du récipient et d'une extrémité proximale **7** dont la fonction apparaîtra, plus précisément, dans la suite de la description. Dans l'exemple illustrée à la **Fig. 6****,** le bouchon **4** du récipient **3** est traversé par l'extrémité distale **6** de l'aiguille creuse **5** à la suite d'un rapprochement relatif entre le récipient **3** et l'aiguille creuse **5.** Bien entendu, il peut être prévu de réaliser un bouchon **4** équipé dès l'origine de l'aiguille creuse **5.**

Conformément à l'invention, le dispositif **1** comporte une chambre de pressurisation **8** d'un fluide contenu dans une telle chambre. Cette chambre de pressurisation **8** est délimitée entre un corps **9** et un piston **11** coopérant de manière étanche avec le corps **9**. Tel que cela ressort des **Fig. 2** et **3****,** le corps **9** se présente sous la forme d'un tube **10** de section droite transversale circulaire, muni à une extrémité, d'un fond **12** et à son extrémité opposée, d'une ouverture **13** pour le passage du piston **11.** Le piston **11** comporte une cloison rigide **14** pourvue ou non d'un joint et présentant une forme complémentaire avec le tube **10** du corps pour coopérer ensemble de manière étanche. Le tube **10** est pourvu au niveau de son fond **12,** d'une cloison de fermeture **15** permettant de délimiter avec la cloison rigide **14** du piston et la paroi du tube **10** située entre ces cloisons, la chambre de pressurisation **8** à volume variable.

Selon une caractéristique préférée de réalisation, le piston **11** est pourvu de l'aiguille creuse **5** dont l'extrémité distale **6** est tournée en direction opposée de la chambre de pressurisation **8** en s'étendant en saillie à partir d'un côté de la cloison rigide **14**. Selon une caractéristique avantageuse de réalisation, la longueur de l'extrémité distale **6** de l'aiguille creuse **5** qui s'étend en saillie à partir de la cloison rigide **14** est suffisante pour traverser le bouchon **4** et déboucher à l'intérieur du récipient **3.** L'extrémité proximale **7** de l'aiguille creuse **5** s'étend en direction de la chambre de pressurisation **8** et en saillie à partir de l'autre côté de la cloison rigide **14.** Ainsi, l'aiguille creuse **5** est fixée de manière étanche par tous moyens appropriés à cette cloison rigide **14** en la traversant de part en part, et en dépassant de part et d'autre de cette cloison,

Selon une autre caractéristique préférée de réalisation, le piston **11** est pourvu d'un manchon de protection **16** entourant à distance, l'extrémité distale **6** de l'aiguille creuse **5**, pour délimiter intérieurement, un volume de réception pour au moins une partie du récipient **3**. Avantageusement, le manchon de protection **16** s'élève à partir de la cloison rigide **14**, sur une hauteur supérieure à la longueur de l'extrémité distale **6** de l'aiguille creuse **5** de manière à éviter toute blessure par cette partie de l'aiguille creuse,

Il ressort de la description qui précède que le piston **11** et le corps **9** sont montés mobiles en translation relativement entre eux selon un sens de rapprochement représenté par la flèche f et un sens d'écartement de sens opposé, représenté par la flèche **f1**. Ainsi, le déplacement du piston **11** selon le sens de rapprochement conduit à diminuer le volume de la chambre **8** et par suite, à augmenter la pression du fluide à l'intérieur de la chambre **8**.

Il est à noter que lors de l'utilisation du dispositif 1 dans laquelle le bouchon **4** du récipient **3** est traversé par l'extrémité distale **6** de l'aiguille creuse **5**, cette chambre de pressurisation **8** communique grâce à l'aiguille creuse **5**, avec l'intérieur du récipient **3** dont la pression présente une valeur identique à celle de la chambre **8**. Aussi, l'augmentation de la pression du fluide à l'intérieur de la chambre **8** entraîne une augmentation correspondante de la pression du fluide à l'intérieur du récipient **3**.

Selon une caractéristique avantageuse de réalisation, le dispositif **1** selon l'invention comporte un système **18** de réglage de la pression de la chambre de pressurisation **8** et par suite, du récipient **3.** Dans l'exemple illustré, le système **18** de réglage de la pression de la chambre de pressurisation **8** est réalisé par l'intermédiaire d'un trou traversant aménagé dans le tube **10** pour mettre à la pression atmosphérique la chambre **8** tant que le piston **11** occupe une position en retrait du trou **18.** Ainsi, dès que la position du piston **11** est telle que la chambre ne communique plus avec le trou **18,** alors la compression du fluide peut intervenir. Ce système **18** permet de régler la pression de la chambre de pressurisation **8** en assurant sa mise à la pression atmosphérique tant que l'écartement entre l'aiguille creuse **5** et le septum **20,** n'atteint pas une valeur déterminée par la position du trou **18.**

Avantageusement, le système **18** permet de régler à une valeur variable, la valeur de la pression de la chambre de pressurisation **8.** Selon un exemple de réalisation, le système **18** comporte un coulisseau monté mobile dans le sens de déplacement du piston et muni d'un trou adapté pour communiquer avec une fente aménagée dans le tube **10** et fermée par le coulisseau à l'exception du trou. La translation du coulisseau permet de régler le positionnement du trou de communication à la pression atmosphérique, et par suite de régler le volume de la chambre **8.** Selon un autre exemple de réalisation, la cloison de fermeture **15** du corps **9** est montée mobile par rapport au tube **10** (par exemple par vissage) pour permettre de faire varier le volume de la chambre **8.**

Selon une caractéristique de l'invention, la chambre de pressurisation **8** est équipée d'un septum **20** positionné pour être pénétrable par l'extrémité proximale **7** de l'aiguille creuse **5** après déplacement de cette dernière selon la direction de rapprochement. A cet effet, la cloison de fermeture **15** du corps **9** est équipé du septum **20** qui se trouve ainsi positionné en face de l'extrémité proximale **7** de l'aiguille creuse **5.** Par exemple, le septum **20** peut être réalisé entre autres par bi-injection, surmoulage ou insertion mécanique après moulage.

Avantageusement, le dispositif **1** selon l'invention comporte un système de guidage en translation de l'extrémité proximale **7** de l'aiguille creuse **5,** en amont du septum **20**. Ce système de guidage non représenté est adapté pour positionner l'aiguille creuse afin que l'extrémité proximale 7 traverse correctement le septum **20.**

Le dispositif **1,** selon l'invention, comporte un système de rapprochement relatif **22** entre l'aiguille creuse **5** et le septum **20** pour assurer selon une première course, une augmentation de la pression du fluide à intérieur de la chambre **8** jusqu'à atteindre une pression de transfert, et dans une deuxième course subséquente à la première, la traversée du septum **20** par l'aiguille creuse **5** afin que l'aiguille creuse **5** débouche dans une chambre de distribution **24** du sang possédant une pression inférieure à la pression de transfert. Cette chambre de distribution **24** est ainsi séparée de la chambre de pressurisation **8**, notamment par le septum **20** qui est en contact d'un côté avec la chambre de distribution **24** et du côté opposé, avec la chambre de pressurisation **8.**

Le système **22** qui est adapté pour rapprocher notamment l'aiguille creuse **5** et le septum **20** exerce un effort de poussée mécanique sur l'aiguille creuse selon le sens **f** et /ou le septum **20** selon le sens **f1.** Selon la variante de réalisation illustrée sur les dessins, le système de rapprochement **22** est du type manuel mais il clair que le déplacement entre l'aiguille creuse **5** et le septum **20** peut être motorisé.

Pour favoriser l'application manuelle d'un effort de poussée, le tube **10** du corps **9** comporte extérieurement, au niveau de son ouverture **13,** deux ailettes **26** de préhension tandis que l'embout de protection **16** est muni, à l'opposé de la cloison rigide **14,** d'une collerette d'appui **27.** Classiquement, un effort de poussée est exercé sur la collerette **27** du manchon du piston **11** tandis que le corps **9** est maintenu en position à l'aide des ailettes **26.** Selon cet exemple de réalisation, un système de visualisation de l'enfoncement du piston **11** dans le corps **9** est prévu pour maîtriser la pression manuelle appliquée. Par exemple, des graduations sont aménagées sur le piston **11** pour visualiser l'enfoncement du piston **11**.

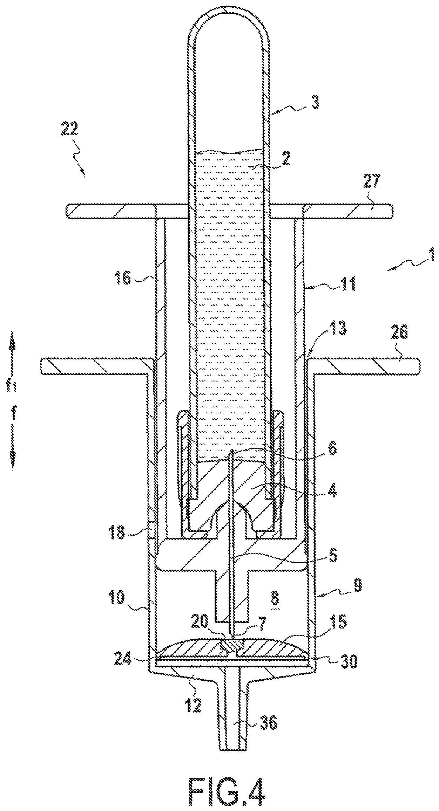

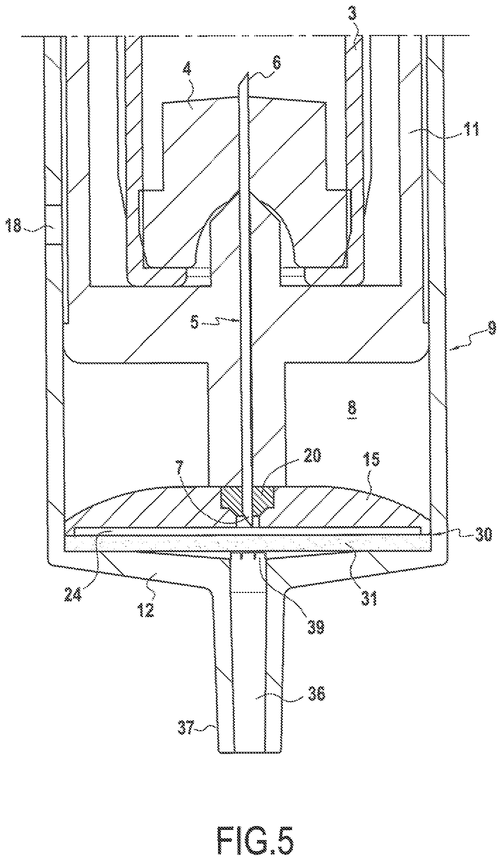

Il ressort de la description qui précède que le piston **11** est apte à occuper :
- une première position illustrée à la **Fig. 3** dans laquelle la chambre **8** et par suite, le récipient **3,** sont à la pression atmosphérique par le trou **18,**
- une deuxième position illustrée à la **Fig. 2** permettant d'augmenter la pression à l'intérieur de la chambre **8** et par suite, du récipient **3** lors du rapprochement entre les cloisons **14** et **15,**
- une troisième position illustrée à la **Fig. 4** pour laquelle l'aiguille creuse **5** est positionnée juste avant de traverser le septum **20,**
- et une quatrième position illustrée à la **Fig. 5** pour laquelle l'aiguille creuse **5** traverse le septum **20** et communique avec la chambre de distribution **24** qui est avantageusement à la pression atmosphérique.

Selon une caractéristique avantageuse de réalisation, le dispositif **1** selon l'Invention comporte une butée de limitation du mouvement de rapprochement des cloisons **14** et **15,** dans une position prédéterminée dans laquelle l'aiguille creuse **5** traverse, par son extrémité proximale **7,** le septum **20** pour déboucher dans la chambre de distribution **24 (****Fig. 5****).** Dans l'exemple illustré **Fig.4****,** l'extrémité inférieure de la cloison **14** vient directement en butée sur la cloison **15.** Un autre système de butée non représentée pourra être réalisé de toute autre manière appropriée, par exemple, par aménagement sur le manchon **16** pour venir en appui sur les oreilles du corps **10.**

Selon une caractéristique avantageuse de réalisation qui sera mieux comprise dans la suite de la description, le système de rapprochement **22** assure également un écartement relatif entre l'aiguille creuse **5** et le septum **20** pénétrable par l'aiguille. Ce système **22** qui est adapté pour écarter l'aiguille creuse **5** du septum **20** exerce un effort de poussée mécanique sur l'aiguille creuse **5** selon le sens **f1** et/ou le septum **20** selon le sens f. Selon la variante de réalisation illustrée sur les dessins, le système d'écartement **22** est du type manuel mais il clair que le déplacement entre l'aiguille creuse **5** et le septum **20** peut être motorisé.

La chambre de distribution **24** est pourvue du système de filtration **30** qui assure l'extraction du plasma à partir du sang transféré dans ladite chambre.

Tel que cela ressort plus précisément de la **Fig. 5****,** le système de filtration **30** comporte le milieu filtrant **31** conforme à l'invention maintenu en position sur une structure de support telle que le fond **12** du corps **9.** Avantageusement, le milieu filtrant **31** est monté à l'intérieur de la chambre de distribution **24** pour s'étendre à distance du septum et en particulier de la cloison de fermeture **15.** Ainsi, la chambre de distribution **24** présente entre la cloison de fermeture **15** et la surface supérieure du milieu filtrant **31,** un volume de récupération du sang sortant de l'extrémité proximale **7** de l'aiguille creuse **5.** Cette chambre de distribution **24** permet d'assurer la répartition du sang sur toute la surface du milieu filtrant **31.**

Avantageusement, le fond **12** du corps **9** sur lequel repose le milieu filtrant **31** est incurvé en direction d'un orifice **36** de sortie pour le plasma filtré, aménagé dans un embout **37** s'étendant en saillie à partir du fond **12.** De préférence, le fond **12** sur lequel repose le milieu filtrant **31** est pourvu de stries ou de nervures **39** sur le fond **12** évitant ainsi le colmatage du milieu filtrant **31** et limitant la perte de charge (**Fig.7**).

Dans l'exemple illustré sur les dessins, la chambre de distribution **24** est délimitée par une partie du corps **9** de réception du piston. Il est à noter qu'il peut être envisagé de réaliser cette chambre **24** par un boîtier constituant le système de filtration et rapporté sur le corps **9.** Un montage amovible de la chambre de distribution **24** peut être prévu, notamment pour récupérer facilement le milieu filtrant, si celui-ci doit être soumis à des analyses ultérieures. Le plus souvent, le dispositif complet se présentera sous la forme d'un consommable jetable, sans qu'une quelconque manipulation du milieu filtrant **31** ne soit opérée, de sorte qu'un tel montage amovible ne sera pas prévu.

La mise en oeuvre du dispositif **1** conforme à l'invention pour transférer une partie d'un échantillon de sang contenu dans un récipient **3** découle directement de la description qui précède.

La méthode de transfert consiste à mettre en communication, à l'aide de l'aiguille creuse **5,** l'intérieur du récipient **3** et la chambre de pressurisation **8.** A cet effet, le récipient **3** est introduit à l'intérieur du manchon **16** jusqu'à ce que l'extrémité distale **6** de l'aiguille creuse **5** traverse le bouchon **4.** Si la chambre de pressurisation **8** est à la pression atmosphérique grâce au trou **18,** alors le récipient **3** est également placé à la pression atmosphérique **(****Fig.** 3).

Le procédé consiste ensuite à rapprocher relativement l'aiguille creuse 5 et le septum **20** pour augmenter la pression à l'intérieur de la chambre **8** et par suite, du récipient **3.** L'application d'un effort de poussée sur le piston **11** conduit à son déplacement selon le sens **f,** jusqu'à une position au-delà du trou **18** dans laquelle la chambre **8** et le récipient **3** forment ensemble une enceinte hermétique. La poursuite du déplacement du piston **11** selon le sens **f** permet d'augmenter la pression du fluide dont une partie est transférée dans le récipient **3** à travers l'aiguille creuse **5.** Le déplacement du piston **11** est réalisé selon une course déterminée de manière que le piston **11** se trouve positionné juste avant le passage de l'aiguille creuse **5** à travers le septum **20.** Dans cette position illustrée à la **Fig. 4****,** la pression à l'intérieur de la chambre **8** et du récipient **3** atteint une valeur de pression souhaitée appelée pression de transfert. Typiquement, la pression de transfert est comprise entre la pression atmosphérique (notamment la pression atmosphérique standard exercée au niveau moyen de la mer qui est de 1,013.10⁵ Pa) et la pression atmosphérique plus 3 bars.

Le procédé consiste ensuite à poursuivre le rapprochement relatif entre l'aiguille creuse **5** et le septum **20** afin que l'extrémité proximale **7** de l'aiguille creuse **5** traverse le septum **20** pour déboucher dans la chambre de distribution **24 (****Fig. 5****).** Dans la mesure où la pression de cette chambre de distribution **24** présente une pression (typiquement atmosphérique) inférieure à la pression de transfert de la chambre **8** et du récipient **3,** une partie du sang du récipient **3** est transférée, sous l'effet de cette différence de pression, dans ladite chambre de distribution **24,** Le sang provenant du récipient **3** passe ainsi à travers l'aiguille creuse **5** et s'écoule à partir de l'extrémité proximale **7,** dans la chambre de distribution **24,** jusqu'à l'équilibre des pressions entre le récipient **3** et la chambre de distribution **24**.

La chambre de distribution **24** étant équipée du système de filtration pour assurer l'extraction du plasma à partir du sang récupéré dans la chambre **24,** le sang traverse le milieu filtrant **31,** en traversant tout d'abord la zone de préfiltration **100,** puis la zone asymétrique **200,** et alors le plasma s'écoule à partir de l'orifice de sortie **36.**

Selon une variante avantageuse de ce procédé d'extraction, cette méthode consiste au terme de l'écoulement du fluide dans la chambre de distribution **24,** à assurer l'écartement relatif entre l'aiguille creuse **5** et le septum **20** de manière à retirer du septum **20,** l'aiguille creuse **5** qui vient communiquer par son extrémité proximale **7,** avec l'intérieur de la chambre de pressurisation **8.** Dans la mesure où le fluide à l'intérieur de la chambre **8** présente une pression supérieure à celle du récipient **3,** une partie de ce fluide passe à travers l'aiguille creuse **5** entraînent le retour de sang contenu dans l'aiguille **5,** à l'intérieur du récipient **3.** Ainsi, le liquide resté éventuellement à l'intérieur de l'aiguille ne peut s'écouler à partir de cette aiguille.

Il est à noter que le dispositif **1** selon l'invention offre l'avantage de pouvoir adapter, par le système **22,** la pression de la chambre de pressurisation **8** en fonction du volume de fluide contenu à l'intérieur du récipient **3,** ce volume de fluide pouvant varier à la suite d'extractions successives ou en fonction des niveaux de remplissage des récipients **3.** Cette régulation peut être réalisée visuellement ou de manière automatique.

Selon un mode particulier de réalisation, le fluide de la chambre de pressurisation **8** est de l'air. Bien entendu, le fluide de la chambre **8** peut être de nature différente tel qu'un sang non miscible avec le liquide contenu dans le récipient **3.** Dans le même sens, le fluide de la chambre de pressurisation **8** peut comporter un milieu de traitement pour le sang contenu dans le récipient **3,** tout en contenant une quantité de gaz suffisante pour autoriser la compression/pressurisation.

Selon une variante de mise en oeuvre du dispositif **1,** une phase d'étalonnage peut être réalisée pour contrôler la pressurisation initiale de la chambre de pressurisation **8.** Préalablement à la mise en communication du récipient **3** avec la chambre de pressurisation **8,** cette phase vise à placer le septum **20** et l'aiguille creuse 5 en position de proximité c'est-à-dire l'aiguille creuse juste avant de traverser le septum **20.** Un fluide de pressurisation est injecté dans la chambre **8** à travers l'aiguille creuse **5** et à partir de son extrémité distale **6** conduisant à positionner le piston mobile **11** à une distance déterminée de la cloison de fermeture **15** pour délimiter une chambre avec un volume de fluide souhaitée.

Il ressort de la description qui précède que le dispositif **1** selon l'invention permet d'extraire en toute sécurité, une partie d'un échantillon de sang contenu dans un récipient **3.** Cette extraction est réalisée de manière simple et peu onéreuse, puisqu'elle procède uniquement d'une poussée mécanique. Le dispositif **1** est de préférence à usage unique pour chaque récipient.

Avantageusement, ce dispositif permet d'extraire directement du plasma à partir d'un tube de prélèvement de sang total.

Des essais ont été réalisés avec un milieu filtrant de section circulaire et de 20 mm de diamètre composé de l'empilement de :
- une zone de préfiltration : 3 filtres en microfibres de verre vendus par la société Ahlström sous la référence 8964, Chacun de ces filtres a une densité de 75 g/m², et une épaisseur de 0,43 mm. Ils sont soumis à un traitement préalable entraînant la fixation d'anticorps anti-hématies réalisé par immersion du filtre dans une solution contenant lesdits anticorps à raison de 0,5 mg/ml en présence de caséine, suivie d'un séchage.
- une zone de porosité asymétrique avec la plus forte porosité au contact du dernier filtre en microfibres de verre : filtre asymétrique en polysulfone vendu par la société Pall appartenant à la gamme Vivid® et de grade GX. L'épaisseur de ce filtre est de 330 µm +/-20 µm.

Des essais avec le milieu filtrant utilisant un dispositif, selon l'invention, ont montré que le dispositif **1** selon l'invention permet d'extraire au moins 200 µl de plasma en moins de 3 minutes, à partir d'un tube de prélèvement équipé d'un septum, contenant un échantillon de sang total. Par ailleurs, il a été possible d'extraire 4 fois de suite 200 µl de plasma à partir d'un même tube de prélèvement de sang de 4 ml, avec 4 dispositifs de filtration **1** successifs. La mise en oeuvre du dispositif **1** permet notamment d'augmenter le volume traité en un temps donné.

Des expériences réalisées avec un milieu filtrant incomplet (zone de préfiltration non pré-traitée avec des réactifs spécifiques des hématies, absence de zone de préfiltration ...) ont montré une altération du plasma en termes de quantité (faible volume) et de qualité (forte hémolyse ou présence d'éléments figurés du sang dont les hématies).

Notamment, à titre de comparaison, des essais réalisés sans traitement préalable des filtres en microfibres de verre ont montré qu'un bouchage intervenait rapidement au niveau du filtre Vivid®, avec la récupération de plasma en faible quantité et très hémolysé. Par ailleurs, des essais réalisés sans filtre Vivid® ont montré qu'ils laissaient passer des éléments figurés du sang dont les hématies,

## Revendications

1. Milieu filtrant (**31**) comprenant deux zones poreuses positionnées sous la forme d'un empilement :
- une première zone poreuse, dite de préfiltration, (**100**) traitée avec au moins un réactif présentant une affinité pour les hématies et entraînant la capture ou l'agglutination des hématies sur la zone de préfiltration,
- une deuxième zone poreuse, dite asymétrique, **(200)** présentant une porosité décroissante transversalement à son épaisseur, la partie de porosité plus élevée étant positionnée du côté de la zone de préfiltration (**100**).

2. Milieu filtrant (**31**) selon la revendication 1 **caractérisé en ce que** la taille minimale des pores de la zone de préfiltration (**100**) est supérieure à la taille maximale des pores de la zone asymétrique (**200**).

3. Milieu filtrant (**31**) selon la revendication 1 ou 2 **caractérisé en ce que** la zone de préfiltration (**100**) présente une taille de pores supérieure à la taille des hématies, et de préférence appartenant à la gamme allant de 10 µm à 1 mm.

4. Milieu filtrant (**31**) selon l'une quelconque des revendications 1 à 3 **caractérisé en ce que** la zone asymétrique (**200**) présente une taille de pores dans sa partie de porosité maximale laissant passer les hématies, par exemple, appartenant à la gamme allant de 10 à 100 µm et dans sa partie de porosité minimale une taille de pores inférieure à la taille des cellules sanguines, et notamment des hématies, et de préférence appartenant à la gamme allant de 0,1 à 10 µm.

5. **-** Milieu filtrant **(31)** selon l'une quelconque des revendications 1 à 4 **caractérisé en ce que** les réactifs présentant une affinité pour les hématies sont des partenaires de liaison spécifiques des hématies, et notamment des anticorps spécifiques des antigènes présents sur les hématies, ou des lectines, comme l'hémaglutinine.

6. **-** Milieu filtrant (**31**) selon l'une quelconque des revendications 1 à 5 **caractérisé en ce que** la zone de préfiltration (**100**) présente une épaisseur de 0,3 à 5 mm, et notamment de 1,2 à 3 mm.

7. Milieu filtrant (**31**) selon l'une quelconque des revendications 1 à 6 **caractérisé en ce que** la zone asymétrique (**200**) présente une épaisseur de 0,1 à 5 mm, et notamment de 0,2 à 3 mm.

8. Milieu filtrant (**31**) selon l'une quelconque des revendications 1 à 7 **caractérisé en ce que** la zone de préfiltration (**100**) a une tension superficielle de mouillage critique comprise entre 50 et 100.10⁻³ N.m⁻¹ et préférentiellement supérieure à 53.10⁻³ N.m⁻¹.

9. Milieu filtrant (**31**) selon l'une quelconque des revendications 1 à 8 **caractérisé en ce que** la zone asymétrique (**200**) a une tension superficielle de mouillage critique comprise entre 50 et 100.10⁻³ N.m⁻¹ et préférentiellement supérieure à 70.10⁻³ N.m⁻¹.

10. Milieu filtrant (**31**) selon l'une quelconque des revendications 1 à 9 **caractérisé en ce que** la zone de préfiltration (**100**) est constituée d'un matériau fibreux, notamment en fibres de verre.

11. Milieu filtrant **(31)** selon l'une quelconque des revendications 1 à 10 **caractérisé en ce que** la zone asymétrique (**200**) est constituée d'un matériau poreux choisi parmi les polymères synthétiques tels que les polysulfones, polycarbonates, polyesters, polyoléfines, polyamines, polyacryliques, polyvinyles et les matériaux inorganiques tels que les verres, céramiques, métaux.

12. Système de filtration (**30**) du sang, et en particulier du sang total, permettant de récupérer du plasma comprenant une chambre intégrant un milieu filtrant (**31**) selon l'une quelconque des revendications précédentes et équipée d'une entrée pour le sang à filtrer positionnée du côté de la zone de préfiltration (**100**) et d'une sortie pour le plasma située du côté de la zone asymétrique **(200),** le milieu filtrant (**31**) étant positionné dans la chambre de manière à ce que le sang circule depuis l'entrée pour traverser transversalement les deux zones du milieu filtrant (**31**), et récupérer le plasma à la sortie.

13. Procédé d'obtention de plasma à partir d'un échantillon de sang, et en particulier d'un échantillon de sang total, mettant en oeuvre le système selon la revendication 12 dans lequel l'échantillon de sang total est introduit dans le système de filtration (**30**) par l'entrée et le plasma récupéré à la sortie.

14. Dispositif (**1**) pour transférer une partie d'un échantillon de sang, et en particulier d'un échantillon de sang total, contenu dans un récipient muni d'un bouchon, et conduisant à la récupération de plasma, **caractérisé en ce qu'**il comprend :
- une aiguille creuse (**5**) pourvue d'une extrémité distale (**6**) de pénétration à travers le bouchon du récipient et d'une extrémité proximale (**7**),
- une chambre (**8**) de pressurisation d'un fluide traversée par l'extrémité proximale (**7**) de l'aiguille creuse de transfert et équipée d'un septum (**20**) positionné pour être pénétrable par l'extrémité proximale (**7**) de l'aiguille après déplacement de cette dernière, cette chambre (**8**) étant étanche et séparée par le septum, d'une chambre de distribution (**24**) du liquide dans laquelle peut déboucher l'extrémité proximale de l'aiguille creuse et
- un système (**22**) de rapprochement relatif entre l'aiguille creuse (**5**) et le septum (**20**) pour assurer une augmentation de la pression du fluide à la suite dudit rapprochement, ce système de rapprochement étant commandé selon une course déterminée pour dans une première phase, augmenter la pression à l'intérieur de la chambre étanche (**8**) jusqu'à atteindre une pression de transfert juste avant le passage de l'aiguille creuse (**5**) à travers le septum (**20**) et dans une deuxième phase, que l'aiguille creuse (**5**) traverse le septum (**20**) pour déboucher dans la chambre (**24**) de distribution du sang possédant une pression inférieure à la pression de transfert afin d'assurer, sous l'effet de cette différence de pression, le transfert d'une partie du sang du récipient (**3**) dans ladite chambre de distribution (**24**), ladite chambre de distribution (**24**) comportant un système de filtration (**30**) selon la revendication 12 permettant d'assurer l'extraction du plasma.

15. Dispositif (**1**) selon la revendication 14, **caractérisé en ce que** le système de rapprochement relatif (**22**) assure également un écartement relatif entre l'aiguille creuse (**5**) et le septum (**20**) pénétrable par aiguille, de manière que l'aiguille creuse communique avec l'intérieur de la chambre de pressurisation (**8**) afin d'assurer, par le fluide sous pression, le retour du sang contenu dans l'aiguille (**5**) à l'Intérieur du récipient (**3**).

16. Dispositif (**1**) selon la revendication 14 ou 15, **caractérisé en ce que** le milieu filtrant (**31**) de la chambre de distribution (**24**) s'étend à distance du septum pénétrable par l'aiguille pour assurer la répartition du sang sur le milieu filtrant (31).

17. Dispositif (**1**) selon l'une quelconque des revendications 14 à 16, **caractérisé en ce que** le milieu filtrant (**31**) est maintenu en position sur une structure de support sur laquelle repose le milieu filtrant (**31**).

18. Dispositif (**1**) selon l'une quelconque des revendications 14 à 17, **caractérisé en ce que** le système de rapprochement (**22**) comporte un corps de réception (**9**) pour un piston (**11**) délimitant entre eux la chambre de pressurisation (**8**), le piston (**11**) et le corps étant mobile en translation relativement entre eux.

19. Dispositif (**1**) selon la revendication 18, **caractérisé en ce que** le piston (**11**) est pourvu de l'aiguille (**5**) dont l'extrémité distale (**6**) est tournée en direction opposée de la chambre de pressurisation (**8**) tandis que l'extrémité proximale (**7**) s'étend à l'intérieur de la chambre de pressurisation (**8**) selon une direction parallèle à la direction de rapprochement et/ou d'écartement.

20. Dispositif **(1)** selon la revendication 19, **caractérisé en ce que** le piston (**11**) est pourvu d'un manchon de projection (**16**) entourant l'extrémité distale (**6**) de l'aiguille (**5**), en délimitant un volume de réception pour au moins une partie du récipient (**3**).

21. Dispositif (**1**) selon la revendication 20, **caractérisé en ce que** le corps (**9**) de réception du piston **(11)** est pourvu au niveau de son fond situé dans le prolongement du piston, du septum **(20)** pénétrable par l'extrémité proximale **(7)** de l'aiguille.

22. Dispositif **(1)** selon l'une quelconque des revendications 18 à 21, **caractérisé en ce que** la chambre **(24)** de distribution du sang est délimitée par une partie du corps de réception du piston ou parlé boîtier muni du système de filtration et rapporté sur le corps.

23. Dispositif **(1)** selon l'une quelconque des revendications 14 à 22, **caractérisé en ce qu'**il comporte un système de guidage en translation de l'extrémité proximale **(7)** de l'aiguille creuse **(5), en** amont du septum **(20).**

24. Dispositif **(1)** selon l'une quelconque des revendications 14 à 23, **caractérisé en ce qu'**il comporte un système **(22)** de réglage de la pression de la chambre de pressurisation **(8)** en fonction du volume de fluide contenu à l'intérieur du récipient **(3).**

25. Dispositif **(1)** selon l'une quelconque des revendications 14 à 24, **caractérisé en ce qu'**il comporte une butée de limitation du mouvement de rapprochement dans une position prédéterminée dans laquelle l'aiguille **(5)** traverse, par son extrémité proximale **(7),** le septum **(20)** pour déboucher dans la chambre **(24)** de distribution du sang.

26. Procédé pour transférer une partie d'un échantillon de sang, et en particulier d'un échantillon de sang total, contenu dans un récipient **(3)** muni d'un bouchon **(4),** permettant de récupérer du plasma, **caractérisé en ce qu'**il consiste:
- à mettre en communication, à l'aide d'une aiguille creuse **(5)** traversant le bouchon **(4)** du récipient, l'intérieur du récipient et une chambre de pressurisation **(8)** d'un fluide, équipée d'un septum **(20)** pénétrable par aiguille et présentant un volume variable à la suite du déplacement relatif entre l'aiguille creuse **(5)** et le septum **(20)** pénétrable par aiguille,
- à assurer le rapprochement relatif entre l'aiguille creuse **(5)** et le septum **(20)** pénétrable par aiguille selon une course déterminée afin d'augmenter la pression à l'intérieur de la chambre **(8)** et par suite du récipient par le transfert du fluide à travers l'aiguille creuse et d'atteindre une pression de transfert juste avant le passage de l'aiguille creuse à travers le septum **(20),**
- à poursuivre le rapprochement relatif entre l'aiguille creuse **(5)** et le septum **(20)** pénétrable par aiguille afin que l'aiguille creuse traverse le septum pour déboucher dans une chambre **(24)** de distribution du sang possédant une pression inférieure à la pression de transfert afin d'assurer, sous l'effet de cette différence de pression, le transfert à travers l'aiguille creuse **(5),** d'une partie du sang du récipient **(3)** dans ladite chambre de distribution **(24),** et
- assurer l'écoulement du sang transféré dans la chambre de distribution **(24),** à travers un système de filtration **(30)** selon la revendication 12 pour assurer l'extraction du plasma.

## Patentansprüche

1. Filtermedium (31), umfassend zwei poröse Zonen, die in Form eines Stapels angeordnet sind:
- eine erste poröse, sogenannte Vorfilterzone (100), die mit mindestens einem Reagens behandelt ist, das eine Affinität für die roten Blutkörperchen aufweist und das Einfangen oder Verklumpen der roten Blutkörperchen auf der Vorfilterzone hervorruft,
- eine zweite poröse, sogenannte asymmetrische Zone (200), die eine quer zu seiner Dicke abnehmende Porosität aufweist, wobei der Teil mit der höchsten Porosität auf der Seite der Vorfilterzone (100) positioniert ist.

2. Filtermedium (31) gemäß Anspruch 1, **dadurch gekennzeichnet, dass** die Mindestgröße der Poren der Vorfilterzone (100) größer als die Maximalgröße der Poren der asymmetrischen Zone (200) ist.

3. Filtermedium (31) gemäß Anspruch 1 oder 2, **dadurch gekennzeichnet, dass** die Vorfilterzone (100) eine größere Porengröße als die Größe der roten Blutkörperchen und vorzugsweise in dem Bereich von 10 µm bis 1 mm aufweist.

4. Filtermedium (31) gemäß einem der Ansprüche 1 bis 3, **dadurch gekennzeichnet, dass** die asymmetrische Zone (200) eine Porengröße in ihrem Teil mit maximaler Porosität aufweist, die die roten Blutkörperchen, die beispielsweise in dem Bereich von 10 bis 100 µm liegen, durchlässt, und in ihrem Teil mit minimaler Porosität eine kleinere Porengröße als die Größe der Blutzellen und insbesondere der roten Blutkörperchen aufweist, die vorzugsweise in dem Bereich von 0,1 bis 10 µm liegt.

5. Filtermedium (31) gemäß einem der Ansprüche 1 bis 4, **dadurch gekennzeichnet, dass** die Reagenzien, die eine Affinität für die roten Blutkörperchen aufweisen, spezifische Bindungspartner der roten Blutkörperchen und insbesondere spezifische Antikörper der auf den roten Blutkörperchen vorhandenen Antigene oder Lectine, wie Hämoglutinin, sind.

6. Filtermedium (31) gemäß einem der Ansprüche 1 bis 5, **dadurch gekennzeichnet, dass** die Vorfilterzone (100) eine Dicke von 0,3 bis 5 mm und insbesondere von 1,2 bis 3 mm aufweist.

7. Filtermedium (31) gemäß einem der Ansprüche 1 bis 6, **dadurch gekennzeichnet, dass** die asymmetrische Zone (200) eine Dicke von 0,1 bis 5 mm und insbesondere von 0,2 bis 3 mm aufweist.

8. Filtermedium (31) gemäß einem der Ansprüche 1 bis 7, **dadurch gekennzeichnet, dass** die Vorfilterzone (100) eine kritische Benetzungsoberflächenspannung zwischen 50 und 100.10⁻³ N.m⁻¹ und vorzugsweise über 53.10⁻³ N.m⁻¹ hat.

9. Filtermedium (1) gemäß einem der Ansprüche 1 bis 8, **dadurch gekennzeichnet, dass** die asymmetrische Zone (200) eine kritische Benetzungsoberflächenspannung zwischen 50 und 100.10⁻³ N.m⁻¹ und vorzugsweise über 70.10⁻³ N.m⁻¹ hat.

10. Filtermedium (31) gemäß einem der Ansprüche 1 bis 9, **dadurch gekennzeichnet, dass** die Vorfilterzone (100) von einem faserigen Material, insbesondere Glasfasern, gebildet ist.

11. Filtermedium (31) gemäß einem der Ansprüche 1 bis 10, **dadurch gekennzeichnet, dass** die asymmetrische Zone (200) von einem porösen Material gebildet ist, das unter den synthetischen Polymeren, wie den Polysulfonen, Polycarbonaten, Polyestern, Polyolefinen, Polyaminen, Polyacrylen, Polyvinylen und den anorganischen Materialien, wie Glas, Keramik, Metall, ausgewählt ist.

12. System (30) zur Filterung des Blutes, und insbesondere des Vollblutes, das es ermöglicht, Plasma wiederzugewinnen, umfassend eine Kammer, die ein Filtermedium (31) gemäß einem der vorhergehenden Ansprüche umfasst und mit einem Eingang für das zu filternde Blut, der auf der Seite der Vorfilterzone (100) angeordnet ist, und einem Ausgang für das Plasma, der auf der Seite der asymmetrischen Zone (200) angeordnet ist, versehen ist, wobei das Filtermedium (31) in der Kammer angeordnet ist, so dass das Blut von dem Eingang aus zirkuliert, um quer durch die zwei Zonen des Filtermediums (31) zu fließen und das Plasma am Ausgang wiederzugewinnen.

13. Verfahren zur Gewinnung von Plasma aus einer Blutprobe und insbesondere einer Vollblutprobe, das das System gemäß Anspruch 12 einsetzt, bei dem die Vollblutprobe in das Filtersystem (30) durch den Eingang eingeführt und das Plasma am Ausgang gewonnen wird.

14. Vorrichtung (1), um einen Teil einer Blutprobe und insbesondere einer Vollblutprobe, die in einem mit einem Stöpsel versehenen Behälter enthalten ist, zu transferieren, wodurch es zu einer Wiedergewinnung von Plasma kommt, **dadurch gekennzeichnet, dass** sie umfasst:
- eine Hohlnadel (5), die mit einem distalen Ende (6) zum Eindringen durch den Stöpsel des Behälters und mit einem proximalen Ende (7) versehen ist,
- eine Kammer (8) zur Druckbeaufschlagung eines Fluids, durch die das proximale Ende (7) der Transferhohlnadel geht, und die mit einem Septum (20) ausgestattet ist, das positioniert ist, um von dem proximalen Ende (7) der Nadel nach Verschiebung dieser letztgenannten durchdrungen zu werden, wobei diese Kammer (8) dicht und durch das Septum von einer Kammer (24) zur Verteilung der Flüssigkeit getrennt ist, in die das proximale Ende der Hohlnadel münden kann, und
- ein System (22) zur relativen Annäherung zwischen der Hohlnadel (5) und dem Septum (20), um eine Erhöhung des Drucks des Fluids nach der Annäherung zu gewährleisten, wobei dieses Annäherungssystem entlang eines bestimmten Weges gesteuert wird, um in einer ersten Phase den Druck in der dichten Kammer (8) zu erhöhen, bis ein Transferdruck direkt vor dem Durchgang der Hohlnadel (5) durch das Septum (20) erreicht wird, und in einer zweiten Phase bis die Hohlnadel (5) durch das Septum (20) hindurchgeht, um in die Kammer (24) zur Verteilung des Blutes zu münden, das einen geringeren Druck als der Transferdruck aufweist, um unter der Wirkung dieses Druckunterschieds den Transfer eines Teils des Blutes von dem Behälter (3) in die Verteilungskammer (24) zu gewährleisten, wobei die Verteilungskammer (24) ein Filtersystem (30) gemäß Anspruch 12 umfasst, das es ermöglicht, die Entnahme des Plasmas zu gewährleisten.

15. Vorrichtung (1) gemäß Anspruch 14, **dadurch gekennzeichnet, dass** das System zur relativen Annäherung (22) auch einen relativen Abstand zwischen der Hohlnadel (5) und dem Septum (20), das von der Nadel durchdringbar ist, gewährleistet, so dass die Hohlnadel mit dem Inneren der Druckbeaufschlagungskammer (8) in Verbindung steht, um durch das Fluid unter Druck die Rückkehr des in der Nadel (5) enthaltenen Blutes in den Behälter (3) zu gewährleisten.

16. Vorrichtung (1) gemäß Anspruch 14 oder 15, **dadurch gekennzeichnet, dass** sich das Filtermedium (31) der Verteilungskammer (24) in einem Abstand zu dem von der Nadel durchdringbaren Septum erstreckt, um die Verteilung des Blutes auf dem Filtermedium (31) zu gewährleisten.

17. Vorrichtung (1) gemäß einem der Ansprüche 14 bis 16, **dadurch gekennzeichnet, dass** das Filtermedium (31) auf einer Tragstruktur, auf der das Filtermedium (31) liegt, in Position gehalten wird.

18. Vorrichtung (1) gemäß einem der Ansprüche 14 bis 17, **dadurch gekennzeichnet, dass** das Annäherungssystem (22) einen Aufnahmekörper (9) für einen Kolben (11) umfasst, der zwischen sich die Druckbeaufschlagungskammer (8) begrenzt, wobei der Kolben (11) und der Körper in Translation in Bezug zueinander beweglich sind.

19. Vorrichtung (1) gemäß Anspruch 18, **dadurch gekennzeichnet, dass** der Kolben (11) mit der Nadel (5) versehen ist, deren distales Ende (6) in die zu der Druckbeaufschlagungskammer (8) entgegengesetzte Richtung gewandt wird, während sich das proximale Ende (7) im Inneren der Druckbeaufschlagungskammer (8) in eine Richtung parallel zur Annäherungs- und/oder Beabstandungsrichtung erstreckt.

20. Vorrichtung (1) gemäß Anspruch 19, **dadurch gekennzeichnet, dass** der Kolben (11) mit einer Schutzhülle (16) versehen ist, die das distale Ende (6) der Nadel (5) umgibt, wobei sie ein Aufnahmevolumen für mindestens einen Teil des Behälters (3) begrenzt.

21. Vorrichtung (1) gemäß Anspruch 20, **dadurch gekennzeichnet, dass** der Aufnahmekörper (9) des Kolbens (11) im Bereich seines Bodens, der sich in der Verlängerung des Kolbens befindet, mit dem Septum (20) versehen ist, das von dem proximalen Ende (7) der Nadel durchdringbar ist.

22. Vorrichtung (1) gemäß einem der Ansprüche 18 bis 21, **dadurch gekennzeichnet, dass** die Kammer (24) zur Verteilung des Blutes durch einen Teil des Aufnahmekörpers des Kolbens oder durch das Gehäuse, das mit dem Filtersystem versehen und auf den Körper aufgesetzt ist, begrenzt ist.

23. Vorrichtung (1) gemäß einem der Ansprüche 14 bis 22, **dadurch gekennzeichnet, dass** sie ein System zur Führung des proximalen Endes (7) der Hohlnadel (5) in Translation stromaufwärts zum Septum (20) umfasst.

24. Vorrichtung (1) gemäß einem der Ansprüche 14 bis 23, **dadurch gekennzeichnet, dass** sie ein System (22) zur Regelung des Drucks in der Druckbeaufschlagungskammer (8) in Abhängigkeit von dem in dem Behälter (3) enthaltenen Fluidvolumen umfasst.

25. Vorrichtung (1) gemäß einem der Ansprüche 14 bis 24, **dadurch gekennzeichnet, dass** sie einen Anschlag zur Begrenzung der Annäherungsbewegung in einer vorbestimmten Position umfasst, in der die Nadel (5) mit ihrem proximalen Ende (7) durch das Septum (20) hindurchgeht, um in der Kammer (24) zur Verteilung des Blutes zu münden.

26. Verfahren, um eine Blutprobe und insbesondere eine Vollblutprobe, die in einem Behälter (3), der mit deinem Stöpsel (4) versehen ist, zu transferieren, wodurch es möglich ist, Plasma wiederzugewinnen, **dadurch gekennzeichnet, dass** es darin besteht:
- mit Hilfe eines Hohlnadel (5), die durch den Stöpsel (4) des Behälters hindurchgeht, das Innere des Behälters und eine Kammer (8) zur Druckbeaufschlagung eines Fluids, die mit einem von der Nadel durchdringbaren Septum (20) ausgestattet ist und ein variables Volumen nach der relativen Verschiebung zwischen der Hohlnadel (5) und dem von der Nadel durchdringbaren Septum (20) aufweist, in Verbindung zu bringen,
- die relative Annäherung zwischen der Hohlnadel (5) und dem von der Nadel durchdringbaren Septum (20) entlang eines vorbestimmten Weges zu gewährleisten, um den Druck in der Kammer (8) und folglich des Behälters durch den Transfer des Fluids durch die Hohlnadel zu erhöhen und einen Transferdruck direkt vor dem Durchgang der Hohlnadel durch das Septum (20) zu erreichen,
- die relative Annäherung zwischen der Hohlnadel (5) und dem von der Nadel durchdringbaren Septum (20) fortzusetzen, damit die Hohlnadel durch das Septum hindurchgeht, um in einer Kammer (24) zur Verteilung des Blutes mit einem geringeren Druck als der Transferdruck zu münden, um unter der Wirkung dieses Druckunterschieds den Transfer eines Teils des Blutes des Behälters (3) durch die Hohlnadel (5) in die Verteilungskammer (24) zu gewährleisten,
- das Fließen des in die Verteilungskammer (24) transferierten Blutes durch ein Filtersystem (30) gemäß Anspruch 12 zu gewährleisten, um die Entnahme des Plasmas zu gewährleisten.

## Claims

1. Filter medium (31) comprising two porous zones arranged in the form of a stack:
a first porous zone (100), named "prefiltration", treated with at least once reagent presenting affinity for red blood cells and leading to red blood cells being captured or agglutinated on the prefiltration zone; and
a second porous zone (200), named "asymmetric", presenting pore size that decreases transversely to its thickness, the portion of higher pore size being positioned towards the prefiltration zone (100).

2. Filter medium (31) according to claim 1, **characterized in that** the minimum pore size in the prefiltration zone (100) is greater than the maximum pore size in the asymmetric zone (200).

3. Filter medium (31) according to claim 1 or claim 2, **characterized in that** the prefiltration zone (100) presents a pore size greater than the size of red blood cells, and preferably lying in the range from 10 µm to 1 mm.

4. Filter medium (31) according to any one of claims 1 to 3, **characterized in that** the asymmetric zone (200) presents a pore size in its portion of maximum pore size that allows red blood cells to pass, for instance lying in the range from 10 µm to 100 µm, and presents a pore size in its portion of minimum pore size that is smaller than the size of blood cells, and in particular red blood cells, and preferably lies in the range from 0.1 µm to 10 µm.

5. Filter medium (31) according to any one of claims 1 to 4, **characterized in that** the reagents presenting affinity for red blood cells are binding partners specific to red blood cells, and in particular antibodies specific to antigens present on red blood cells or lectins such as hemaglutinin.

6. Filter medium (31) according to any one of claims 1 to 5, **characterized in that** the prefiltration zone (100) presents a thickness from 0.3 mm to 5 mm, and in particular from 1.2 mm to 3 mm.

7. Filter medium (31) according to any one of claims 1 to 6, **characterized in that** the asymmetric zone (200) presents a thickness from 0.1 mm to 5 mm, and in particular from 0.2 mm to 3 mm.

8. Filter medium (31) according to any one of claims 1 to 7, **characterized in that** the prefiltration zone (100) has a critical wetting surface tension between 50 and 100×10⁻³ N.m⁻¹, and preferably greater than 53×10⁻³ N.m⁻¹.

9. Filter medium (31) according to any one of claims 1 to 8, **characterized in that** the asymmetric zone (200) has a critical wetting surface tension between 50 and 100×10⁻³ N.m⁻¹, and preferably greater than 70×10⁻³ N.m⁻¹.

10. Filter medium (31) according to any one of claims 1 to 9, **characterized in that** the prefiltration zone (100) is constituted by a fiber material, in particular made of glass fibers.

11. Filter medium (31) according to any one of claims 1 to 10, **characterized in that** the asymmetric zone (200) is constituted by a porous material selected from synthetic polymers such as polysulfones, polycarbonates, polyesters, polyolefins, polyamines, polyacrylics, polyvinyls, and from inorganic materials such as glasses, ceramics, metals.

12. Filtration system (30) for filtering blood, in particular whole blood, enabling plasma to be recovered, comprising a chamber containing a filter medium (31) according to any preceding claim and provided with an inlet for the blood to be filtered that is positioned towards the prefiltration zone (100), and with an outlet for plasma that is situated towards the asymmetric zone (200), the filter medium (31) being positioned in the chamber in such a manner that the blood flow from the inlet to cross through both zones of the filter medium (31), with plasma being recovered at the outlet.

13. Process for obtaining plasma from a blood sample, and in particular a sample of whole blood, the process implementing the system according to claim 12 in which the sample of hole blood is introduced into the filtration system (30) via the inlet and plasma is recovered from the outlet.

14. Device (1) for transferring a portion of a blood sample, and in particular a sample of whole blood, contained in a container provided with a plug, and leading to plasma being recovered, the device being **characterized in that** it comprises:
a hollow needle (5) provided with a distal end (6) for penetrating through the plug of the container and with a proximale end (7);
a pressure chamber (8) for pressurizing a fluid and having the proximal end (7) of the hollow transfer needle passing therethrough, the chamber being provided with a septum (20) positioned to be penetrable by the proximal end (7) of the needle after the needle has moved, said chamber (8) being leaktight and separated by the septum from a liquid distribution chamber (24) into which the proximal end of the hollow needle can open out; and
an approach system (22) for causing the hollow needle (5) and the septum (20) to approach each other so as to increase the pressure of the fluid as a result of said approach, the approach system being controlled to move over a determined stroke so that in a first stage the pressure inside the leaktight chamber (8) is increased up to a transfer pressure immediately prior to the hollow needle (5) passing through the septum (20), and in a second stage the hollow needle (5) passes trough the septum (20) to open out intro the blood distribution chamber (24) that is at a pressure lower than the transfer pressure so that, under the effect of this pressure difference, a portion of the blood is transferred from the container (3) into said distribution chamber (24), said distribution chamber (24) including a filtration system (30) according to claim 12 for extracting plasma.

15. Device (1) according to claim 14, **characterized in that** the approach system (22) also enables the hollow needle (5) and the septum (20) that is penetrable by needle to be separated relative to each other in such a manner that the hollow needle communicates with the inside of the pressure chamber (8) so as to enable the fluid under pressure to cause the blood contained in the needle (5) to be returned intro the container (3).

16. Device (1) according to claim 14 or claim 15, **characterized in that** the filter medium (31) in the distribution chamber (24) lies at a distance from the septum that is penetrable by needle so as to ensure that the blood is distributed over the filter medium (31).

17. Device (1) according to any one of claims 14 to 16, **characterized in that** the filter medium (31) is held in position on a support structure on which the filter medium (31) rests.

18. Device (1) according to any one of claims 14 to 17, **characterized in that** the approach system (22) comprise a receiving body (9) for receiving a piston (11) together defining the pressure chamber (8), the piston (11) and the receiving body being movable in translation relative to each other.

19. Device (1) according to claim 18, **characterized in that** the piston (11) is provided with a needle (5) having its distal end (6) pointing away from the pressure chamber (8) and its proximal end (7) extending inside the pressure chamber (8) in a direction parallel to the approach and/or séparation direction.

20. Device (1) according to claim 19, **characterized in that** the piston (11) is provided with a projective sleeve (16) surrounding the distal end (6) of the needle (5) and defining a volume for receiving at least a portion of the container (3).

21. Device (1) according to claim 20, **characterized in that** the receiving boy (9) for receiving the piston (11) is provided at its end well situated in line with the piston with the septum (20) that can be penetrated by the proximal end (7) of the needle.

22. Device (1) according to any one of claims 18 to 21, **characterized in that** the blood distribution chamber (24) is defined by a portion of the receiving body for receiving the piston or by the separate unit containing the filtration system and fitted to the receiving body.

23. Device (1) according two any one of claims 14 to 22, **characterized in that** it includes a system for guiding movement in translation of the proximal end (7) of the hollow needle (5) upstream from the septum (20).

24. Device (1) according to any one of claims 14 to 23, **characterized in that** it includes a system (22) for adjusting the pressure of the pressure chamber (8) as a function of the volume of fluid contained inside the container (3).

25. Device (1) according to any one of claims 14 to 24, **characterized in that** it includes an abutment for limiting the approach movement in a predetermined position in which the proximal end (7) of the needle (5) passes through the septum (20) to open out into the blood distribution chamber (24).

26. Process for transferring a portion of a blood sample, and in particular a sample of thole blood, contained in a container (3) provided with a plug (4), the process enabling plasma to be recovered and being **characterized in that** it consists in:
using a hollow needle (5) passing through the plug (4) of the container to put the inside of the container into communication with a pressure chamber (8) for pressurizing a fluid, said pressure chamber (8) being provided with a septum (20) that is penetrable by needle and presenting a volume that is variable as a result of relative movement between the hollow needle (5) and the septum (20) that is penetrable by needle;
causing the hollow needle (5) and the septum (20) that is penetrable by needle to approach each other over a determined stroke so as to increase the pressure inside the chamber (8) and consequently inside the container by transferring the fluid via the hollow needle and reaching a transfer pressure immediately prior to the hollow needle passing through the septum (20);
continuing to cause the hollow needle (5) and the septum (20) that is penetrable by needle to approach each other so that the hollow needle passes through the septum so as to open out into a blood distribution chamber (24) having a pressure that is lower than the transfer pressure so that under the effect of this pressure difference a portion of the blood is transferred through the hollow needle (5) from the container (3) intro said distribution chamber (24); and
ensuring that the blood transferred into the distribution chamber (24) flows through a filtration system (30) according to claim 12 in order to extract plasma.
